(19) Europäisches Patentamt
European Patent Office
Office européen des brevets

(11) Veröffentlichungsnummer: **0 287 884**
**B1**

(12) # EUROPÄISCHE PATENTSCHRIFT

(45) Veröffentlichungstag der Patentschrift: **09.01.91**

(51) Int. Cl.$^5$: **C 07 C 47/127,** C 07 C 45/37

(21) Anmeldenummer: **88105374.8**

(22) Anmeldetag: **02.04.88**

(54) **Verfahren zur Herstellung von Carbonylverbindungen.**

(30) Priorität: **15.04.87 DE 3712856**

(43) Veröffentlichungstag der Anmeldung:
**26.10.88 Patentblatt 88/43**

(45) Bekanntmachung des Hinweises auf die Patenterteilung:
**09.01.91 Patentblatt 91/02**

(84) Benannte Vertragsstaaten:
**BE DE FR GB IT NL**

(56) Entgegenhaltungen:
**EP-A-0 007 570**
**DE-A-1 923 048**
**DE-A-2 158 343**
**DE-C-1 967 147**
**US-A-4 258 216**
**US-A-4 503 261**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Fritz, Graf, Dr.**
**Im Rothschild 33**
**D-6720 Speyer (DE)**
Erfinder: **Hupfer, Leopold, Dr.**
**Waltershoehe 3**
**D-6701 Friedelsheim (DE)**
Erfinder: **Schultheiss, Harald, Dr.**
**An der Adamslust 9**
**D-6710 Frankenthal (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Diese Erfindung betrifft ein neues Verfahren zur Herstellung von Carbonylverbindungen durch Gasphasenoxidation von Alkoholen mit einem Sauerstoff enthaltenden Gas in Gegenwart von Kupfer und Silber enthaltenden Katalysatoren.

Aus den deutschen Patentschriften 19 23 048 und 19 67 147 ist bekannt, daß man Glyoxal durch Gasphasenoxidation von Ethylenglykol an einem Oxidationskatalysator herstellen kann, der Kupfer in Kombination mit Zinn, Phosphor, Arsen, Antimon oder Wismut oder Silber in Kombination mit Phosphor enthält. Nach diesen Verfahren werden hohe Glyoxalausbeuten nur bei unvollständigem Umsatz, d.h. bei hohen Restglykolgehalten erzielt. Außerdem sinken die Ausbeuten bei längerer Betriebsdauer, weshalb man die Katalysatoren durch aufwendige Verfahren regenerieren muß (DE—OS 21 58 343).

Nach dem Verfahren der EP—PS 7 570 erhält man bei der Gasphasenoxidation von Ethylenglykol an Kupferkatalysatoren vorteilhafte Ergebnisse hinsichtlich der Lebensdauer der Katalysatoren und der Glyoxalausbeute, wenn die Reaktion in Gegenwart einer flüchtigen Phosphorverbindung durchführt. Es hat sich jedoch bei längerer Betriebsdauer herausgestellt, daß die Glyoxalausbeute und Produktreinheit mit der Versuchsdauer zunehmend schlechter werden. Eine unbefriedigende Produktqualität, die insbesondere auf einen hohen Restgehalt an Glykolaldewhyd zurückzuführen ist, wird auch bei Verwendung von Katalysatorschichten erhalten, die aus Kupfer- und Silberkristallen bestehen. Bei dieser Methode (US—PS 4 503 261) wird das Ethylenglykol bei hohen Temperaturen (450 bis 800°C) und kurzen Verweilzeiten (nicht höher als 0,05 sec ) umgesetzt. Anteile von Glykol und Nebenprodukten, wie Glykolaldehyd und Formaldehyd im Glyoxal sind für viele Anwendungszwecke des Glyoxals höchst unerwünscht. Da eine nachträgliche Entfernung von Glykol und Glykolaldehyd aus Glyoxallösungen mit vertretbarem wirtschaftlichem Aufwand nicht möglich ist, war nach einem Verfahren zu suchen, das es gestattet, Glyoxal durch katalytische Gasphasenoxidation von Ethylenglykol auch bei langen Betriebszeiten unter weitgehender Vermeidung der Bildung störender Nebenprodukte herzustellen.

Es wurde nun gefunden, daß man bei der Herstellung von Carbonylverbindungen der Formel

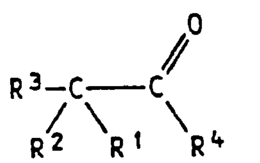

I,

in der $R^1$ ein Wasserstoffatom oder zusammen mit $R^2$ ein Sauerstoffatom, $R^2$ den Rest $OR^5$ oder zusammen mit $R^1$ ein Sauerstoffatom, $R^3$ ein Wasserstoffatom, einen Alkylrest mit 1 bis 8 C-Atomen oder einen Cycloalkylrest, $R^4$ ein Wasserstoffatom oder einen Alkylrest mit 1 bis 3 C-Atomen und $R^5$ eine Cycloalkylrest, einen Alkylrest mit 1 bis 4 C-Atomen oder einen Alkoxyalkylrest, die eine Keto- oder Aldehydgruppe enthalten können bedeuten, durch Gasphasenoxidation von Alkoholen der Formel

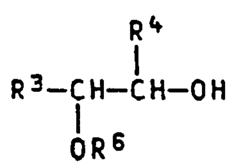

II.

in der $R^3$ und $R^4$ die oben angegebene Bedeutung haben und R ein Wasserstoffatomen, einen Alkyl- oder Hydroxyalkylrest mit 1 bis 4 C-Atomen, einen Cycloalkylrest oder einen Alkoxyalkylrest, der eine Hydroxylgruppe enthalten kann, bedeutet, mit einem Sauerstoff enthaltenden Gas in Gegenwart von Kupfer und Silber enthaltenden Katalysatoren auf besonders vorteilhafte Weise herstellen kann, wenn man das gasförmige Ausgangsgemisch bei Temperaturen von 300 bis 400°C zuerst über einen Kupferkatalysator, dessen aktive Masse einen Kupfergehalt von mehr als 90 Gew.% aufweist, und danach über einen Silberkatalysator, dessen aktive Masse einen Silbergehalt von mehr als 90 Gew.% aufweist, leitet, wobei die Verweilzeit der Reaktionsgase im Reaktor 0,5 bis 3 Sekunden beträgt.

Nach dem neuen Verfahren erhält man z.B. das Glyoxal aus Ethylenglykol auch im Dauerbetrieb in hoher Ausbeute und Reinheit.

In den Alkoholen der Formel II bedeuten Alkylreste z.B. Methyl-, Ethyl-, Propyl- oder Butylreste und Cycloalkylreste z.B. Cyclohexyl- oder Cyclopentylreste. Alkoxyalkylgruppen, die eine Hydroxylgruppe enthalten können, sind z.B. $—CH_2—CH_2—O—CH_2—CH_2—OH—$Reste. Beim dem erfindungsgemäßen Verfahren gehen die endständigen Hydroxylgruppen in Aldehydgruppen und die sekundären Hydroxylgruppen in Ketogruppen über.

Beispielsweise seien die folgenden Ausgangsverbindungen der Formel II genannt:

$HO-CH_2-CH_2-OH$, $H_3COCH_2-CH_2OH$, $C_2H_5OCH_2-CH_2OH$,

$H_3C-CH(OH)-CH_2-OH$, $C_2H_5-CH(OH)-CH_2OH$, 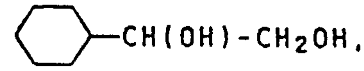,

$HO-(CH_2)_2-O-(CH_2)_2-OH$, 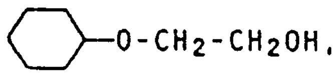, 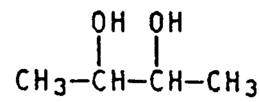

Die Gasphasenoxidation des Alkohols mit dem Sauerstoff enthaltenden Gas an den Katalysatoren führt man bei Temperaturen von 300 bis 400°C durch.

Als Katalysatoren werden metallisches Kupfer und Silber oder Legierungen verwendet, die zu über 90 Gew.% an Kupfer bzw. Silber bestehen. Man wendet sie z.B. in Form von Drehspänen, Draftgeweben, Gazen oder auch als Trägerkatalysatoren mit einem z.B. overflächenarmen, inerten Träger an. Zweckmäßigerweise werden Schalen-Katalysatoren eingesetzt, bei denen die aus den genannten Metallen bestehende aktive Masse als Schale auf einem inerten Formkörper vorliegt. Diese Katalysatoren können auf einfacher Weise durch Beschichten von inertem Trägermaterial, wie α-Aluminiumoxid, Siliciumcarbid oder Seatit mit den Metallen nach an sich bekanntem Verfahren, wie Flammspritzen, Plasmaspritzen und Aufdampfen, hergestellt werden. Besonders geeignete Schalenkatalysatoren bestehen z.B. aus einem Träger aus Steatit, der z.B. die Form von Kugeln, Ringen oder Halbringen aufweist, mit einer darauf angebrachten katalytischen Masse aus Kupfer bzw. Silber, die z.B. 0,1 bis 10 Gew.%, bezogen auf den fertigen Schalen-Katalysator, ausmacht.

Überraschenderweise werden die vorteilhaften Ergebnisse des erfindungsgemäßen Verfahrens bereits durch eine Kombination eines massiven Formkörpers aus reinem Kupfer, das keine Zusatzkomponente enthält, mit einem Silber-Schalenkatalysator der genannten Art erhalten.

Nach einer vorteilhaften Ausführungsform der Erfindung führt man die Gasphasenoxdiation in Gegenwart einer unter den Reaktionsbedinungen flüchtigen Phosphorverbindung durch.

Als unter den Reaktionsbedinungen flüchtige Phosphorverbindungen verwendet man zweckmäßigerweise P-Verbindungen, die unzersetzbar verdampfbar sind unt mit den Komponenten des Synthesegases bei den Umsetzungsbedingungen keine Reaktion eingehen. Das sind z.B. Ester der Phosphorsäure, der phosphorigen Säure oder von Phosphonsäuren, wie Trimethylphosphat, Triethylphosphat, Tri-isopropylphosphat, Tri-n-propylphosphat. Trimethylphosphit, Triethylphosphit, Triethylphosphinoxid, Methylphosphonsäurediethylester, Methylphosphonsäuredimethylester oder Ethylphosphonsäurediethylester. Die Phosphorverbindungen werden so eingesetzt, daß die zugegebene Phosphormenge (berechnet P), bezogen auf die Gewichtsmenge an dem Alkohol 0,01 bis 10 ppm beträgt.

Das Verfahren wird z.B. so ausgeführt, daß man ein gasförmiges Gemisch aus dem Alkohol und Sauerstoff (in einer Menge zwischen ca. 0,05 und 2 Mol, bezogen auf 1 Mol des eingesetzten Alkohols), eventuell zusammen mit Stickstoff (0 bis 99 Vol.% des Gesamtgasgemisches) über den auf 225 bis 500°C gehaltenen Katalysator leitet, wobei man gegebenenfalls die flüchtige Phosphorverbindung dem gasförmigen Ausgangsgemisch vorher zusetzt. Die Verweilzeit der Reaktionsgase im Reaktor beträgt 0,5 bis 3 Sekunden. Das gasförmige Ausgangsgemisch wird dabei zuerst über den Kupferkatalysator und dann über den Silberkatalysator geleitet. Man erreicht dies, indem der erste Teil der Katalysatorbeschickung des Reaktors in Strömungsrichtung aus dem Kupferkatalysator besteht, an den sich dann die aus dem Silberkatalysator bestehende Katalysatorschicht anschließt.

Das den Reaktor verlassende gasförmige Reaktonsgemisch wird wie üblich mit Wasser ausgewaschen. Zur besseren Kontrolle der zugegebenen Phosphormenge im Spurenbereich kann man zweckmäßigerweise auch so vorgehen, daß man die Phosphorverbindung in Wasser oder dem eingesetzten Alkohol gelöst in den heißen Synthesegasstrom eindosiert. Das nach dem erfindungsgemäßen Verfahren aus Ethylenglykol erhaltene Glyoxal, kann man direkt in der Handelsüblichen Form einer 40%igen wäßrigen Lösung erhalten.

Nach dem Verfahren der Erfindung erhält man die Carbonylverbindungen, insbesondere die Aldehyde Glyoxal und Methlyglyoxal, in ausgezeichneter Reinheit bei gleichzeitig recht guter Ausbeute. Es ist überraschend, daß man z.B. bei vergleichsweise niedrigen Temperaturen von etwa 360°C und bei Verwendung von reinem Kupfer und Silber Glyoxal mit so ausgezeichneter Produktqualität erhält. Der Zusatz flüchtiger Phosphorverbindungen ergibt keine Ausbeuteverbesserung, vermindert aber den Restgehalt an Glykolaldehyd.

## Beispiel 1

In ein Reaktorrohr mit der Länge von 50 cm und einem Innendurchmesser von 20 mm wurden 33 ml eines Katalysators aus Kupfer-Drehspänen eingefüllt. Die Kupfer-Drehspäne bestanden aus einem

phosphorfreien Kupfer mit einer Reinheit von mehr als 99,9%. Dann wurden in das Reaktionsrohr 67 ml des nach dem folgenden Absatz hergestellten Silberschalenkatalysators eingebaut. Die Anordnung der beiden Katalysator-Schichten wurden so gewählt, daß der Kupferkatalysator auf der Gaseintrittsseite lag. Man leitete nun pro Stunde ein Gemisch aus 12,4 g Ethylenglykol, 27 N1 Luft sowie 100 N1 Stickstoff durch den Reaktor. Die Reaktortemperatur wurde mit Hilfe einer Salzschmelze auf 360°C eingestellt. Das Reaktionsgas wurde nach Verlassen des Reaktors mit Wasser in Beruhrung gebracht, wobei die Reaktionsprodukte in der wäßrigen Phase gelöst wurden. Die bei der Reaktion entstandenden Permanentgase CO und $CO_2$ verblieben im Abgas und wurden in der Gasphase analysiert. Man erhielt eine Glyoxalausbeute von 71,5 Mol.% bei einem Glykolumsatz von 99 Mol.%. Glykolaldehyd entstand mit einer Ausbeute von lediglich 0,3 Mol.%. Formaldehyd und Säuren wurden mit Ausbeuten von 0,8 und 2,0 Mol.% gebildet. Die Totalverbrennung zu CO und $CO_2$ betrug 24,4 Mol.%. Diese Ergebnisse entsprechen einer Konzentration von Glykol und Glykolaldehyd in der handelsüblichen 40%igen wäßrigen Glyoxallösung von 0,6 und 0,2 Gew.%. Die Formaldehydkonzentration beträgt dabei 0,46 Gew.%.

0,5 l Kugeln aus Steatit mit einem Durchmesser von 3,5 bis 4,5 mm wurden in eine offene Drehtrommel gegeben und mit Silberpulver der Korngröße <60 Mikrometer nach der Methode des Flammspritzens beschichtet. Dabei wurde das Silberpulver in eine Sauerstoff/Acetylen-Flamme eingebracht und mit den Flammengasen in teilweise geschmolzener Form auf den Träger transportiert. Die Silbermenge wurde so bemessen, daß der fertige Katalysator 4 Gew.% Silber bezogen auf die Gesamtmasse enthielt. Die Temperaturen im zu beschichtenden Gut betrugen 400 bis 600°C, die Dauer der Beschichtung war 20 Minuten.

Beispiel 2 (Vergleich: nur Silber)

100 ml des in Beispiel 1 verwendeten Silber-Schalenkatalysators wurden in den in Beispiel 1 beschriebenen Rohrreaktor eingebaut. Nun wurde pro Stunde ein Gemisch aus 6,2 g Ethylenglykol, 13,5 N1 Luft sowie 100 N1 Stickstoff durch den Rohrreaktor geleitet. Die Reaktortemperatur wurde mit Hilfe eine Salzschmelze auf 360°C eingestellt. Die Produktaufarbeitung und Gasanalytik erfolgte wie in Beispiel 1 beschrieben. Man erhielt eine Glyoxalausbeute von 70,6 Mol.% bein einem Umsatz von 97,4 Mol.%. Die Verbrennung zu CO und $CO_2$ betrug 22,9 Mol.%. Glykolaldehyd, Formaldehyd und Säuren wurden zu 1,4 Mol.%, 0,9 Mol.% und 1,6 Mol.% gebildet. Diese Ergebnisse entsprechen einer Konzentration von Glykol und Glykolaldehyd in der handelsüblichen 40%igen wäßrigen Glyoxallösung von 1,6 und 0,9 Gew.%. Die Formaldehydkonzentration beträgt dabei 0,53 Gew.%

Beispiel 3 (Vergleich: nur Kupfer)

100 ml eines Katalysators aus massivem, phosphorfreien Kupfer in der Form von Raschigringen mit den Abmessungen 5 × 5 × 0,3 mm wurden in einen Rohrreaktor mit innerem Durchmesser von 20 mm eingebaut. Man leitete pro Stunde ein Gemisch aus 12,4 g Ethylenglykol, 27 N1 Luft sowie 100 N1 Stickstoff durch den Reaktor. Die Reaktortemperatur wurde mit Hilfe einer Salzschmelze auf 350°C eingestellt. Die Produktaufarbeitung und Gasanalytik erfolgt wie in Beiepisle 1 beschrieben. Man erhielt eine Glyoxalausbeute von 49,7 Mol.%, bei einem Umsatz von 89,8 Mol.%. Die Verbrennung zu CO und $CO_2$ betrug 29,1 Mol.%. Glykolaldehyd, Formaldehyd und Säuren wurden zu 1,0 Mol.%, 8,0 Mol.% und 2,0 Mol.% gebildet. Diese Ergebnisse entsprechen einer Konzentration von Glykol und Glykolaldehyd in der handelsüblichen 40%igen wäßrigen Glyoxallösung von 9,0 und 0,9 Gew.%. Die Formaldehydkonzentration beträgt dabei 6,9 Gew.%.

Beispiel 4

Die Versuchsbedinungen bezüglich Katalysator und Reaktionsbedinungen waren die gleichen wie in Beispiel 1, wobei jedoch zum Synthesegas Trimethylphosphat in einer Menge von 0,33 ppm (berechnet P) bezogen auf die eingsetzte Menge an Ethylenglykol, zudosiert wurde. Man erhielt eine Glyoxalausbeute von 71,2 Mol.%, bei einem Umsatz von 98,7 Mol.%. Die Verbrennung zu CO und $CO_2$ betrug 24,6 Mol.%. Glykolaldehyd, Formaldehyd und Säuren wurden zu 0,2 Mol.%, 0,9 Mol.% und 1,8 Mol % gebildet. Diese Ergebnisse entsprechen einer Konzentration von Glykol und Glykolaldehyd in der handelsüblichen 40%-igen wäßrigen Glyoxallösung von 0,8 und 0,1 Gew.%. Die Formaldehydkonzentration beträgt dabei 0,53 Gew.%

**Patentansprüche**

1. Verfahren zur Herstellung von Carbonylverbindungen der Formel

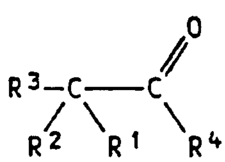

I,

in der $R^1$ eine Wasserstoffatom oder zusammen mit $R^2$ ein Sauerstoffatom, $R^2$ den Rest $OR^2$ oder zusammen mit $R^1$ ein Sauerstoffatom, $R^3$ ein Wasserstoffatom, einen Alkylrest mit 1 bis 8 C-Atomen oder einen Cycloalkylrest, $R^4$ ein Wasserstoffatom oder einen Alkylrest mit 1 bis 3 C-Atomen, und $R^5$ einen Cycloalkylrest, einen Alkylrest mit 1 bis 4 C-Atomen oder einen Alkoxyalkylrest, die eine Keto- oder Aldehydgruppe enthalten können, bedeuten, durch Gasphasenoxidation von Alkoholen der Formel

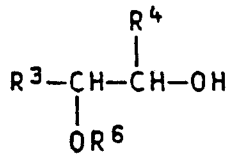

II,

in der $R^3$ und $R^4$ die oben angegebene Bedeutung haben und $R^6$ ein Wasserstoffatom, einen Alkyl- oder Hydroxyalkylrest mit 1 bis 4 C-Atomen, einen Cycloalkylrest oder einen Alkoxyalkylrest, der eine Hydroxylgruppe enthalten kann, bedeutet, mit einem Sauerstoff enthaltenden Gas in Gegenwart von Kupfer und Silber enthaltenden Katalysatoren, dadurch gekennzeichnet, daß man das gasförmige Ausgangsgemisch bei Temperturen von 300 bis 400°C zuerst über einen Kupferkatalysator, dessen aktive Masse einen Kupfergehalt von mehr als 90 Gew.% aufweist, und danach über einen Silberkatalystor, dessen aktive Masse einen Silbergehalt von mehr als 90 Gew.% aufweist, leitet, wobei die Verweilzeit der Reaktionsgase in Reaktor 0,5 bis 3 Sekunden beträgt.

2. Verfahren nach Anspruch 1, dadurch gekennzeichnet, daß man als Alkohol der Formel II eine der Verbindungen der Formeln

$HOCH_2-CH_2OH$, $H_3COCH_2-CH_2OH$, $C_2H_5OCH_2-CH_2OH$,

$H_3C-CH(OH)CH_2-OH$, $C_2H_5-CH(OH)-CH_2OH$, 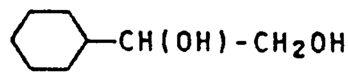,

$HO-(CH_2)_2-O-(CH_2)_2-OH$, 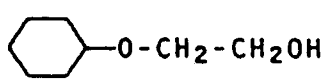, 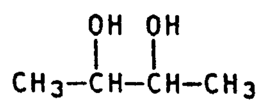

verwendet.

3. Verfahren nach Anspruch 1, dadurch gekennzeichnet, daß man Glyoxal aus Ethylenglykol herstellt.

4. Verfahren nach Anspruch 1, dadurch gekennzeichnet, daß man die Gasphasenoxidation in Gegenwart einer bei den Reaktionsbedigungen flüchtigen Phosphorverbindung durchführt, wobei die Phosphormenge (berechnet P), bezogen auf die Gewichtsmenge an dem Alkohol, 0,01 bis 10 ppm beträgt.

5. Verfahren nach Anspruch 1, dadurch gekennzeichnet, daß man als Kupferkatalysator Formkörper aus Kupfer mit eine Kupfergehalt von mindestens 90 Gew.% einsetzt.

6. Verfahren nach Anspruch 1, dadurch gekennzeichnet, daß man als Kupferkatalysator einen Schalenkatalysator aus einem inerten Trägmaterial und der darauf angebrachten aktiven Masse, die zu mehr als 90 Gew.% aus Kupfer besteht, verwendet.

7. Verfahren nach Anspruch 1, dadurch gekennzeichnet, daß man als Silberkatalysator einen Schalenkatalysator aus einem inerten Trägmaterial und der darauf aufgebrachten aktiven Masse, die zu mehr als 90 Gew.% zus Silber besteht, verwendet.

**Revendications**

1. Procédé de préparation de composés carbonyles de la formule

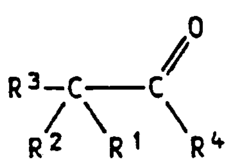

I,

dans laquelle $R^1$ représente un atome d'hydrogène ou, réuni à $R^2$, un atome d'oxygène, $R^2$, le reste $OR^5$ ou, réuni à $R^1$, un atome oxygène, $R^3$, un atome d'hydrogène, un reste alkyle de 1 à 8 atomes C ou un reste cycloalkyle, $R^4$, un atome d'hydrogène ou un reste alkyle de 1 à 3 atomes C et $R^5$ un reste alkyle de 1 à 4

atomes C, un reste cycloalkyle ou un reste alcoxyalkyle qui peut contenir un groupe ceto ou aldéhyde, par oxydation en phase gazeuse d'alcools de la formule

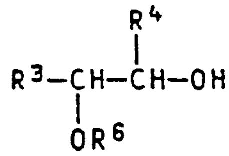

II,

dans laquelle $R^3$ et $R^4$ ont les significations sus-indiquées et $R^6$ représente un atome d'hydrogène, un reste alkyl- ou hydroxyalkyle de 1 à 4 atomes C, un reste alcoxyalkyle qui peut contenir un groupe hydroxyle, avec un gaz contenant de l'oxygène, en présence de catalyseurs contenant du cuivre et de l'argent, caractérisé par le fait que l'on conduit le mélange de départ, à des températures de 300 à 400 degrés C, d'abord sur un catalyseur au cuivre, dont la masse active a une teneur en cuivre de plus de 90% en poids, et puis sur un catalyseur à l'argent, dont la masse active a une teneur en argent de plus de 90% en poids la durée de séjour des gaz de réaction dans le réacteur étant de 0,5 à 3 secondes.

2. Procédé selon la revendication 1, caractérisé par le fait que l'on utilise, comme composés de départ de la formule II, les composés suivants

$HOCH_2-CH_2OH$, $H_3COCH_2-CH_2OH$, $C_2H_5OCH_2-CH_2OH$,

$H_3C-CH(OH)CH_2-OH$, $C_2H_5-CH(OH)-CH_2OH$, 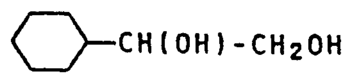,

$HO-(CH_2)_2-O-(CH_2)_2-OH$, 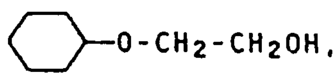, 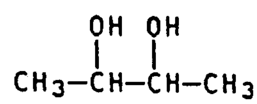

3. Procédé selon la revendication 1, caractérisé par le fait que l'on prépare le glyoxal à partir d'éthylèneglycol.

4. Procédé selon la revendication 1, caractérisé par le fait que l'on effectue l'oxydation en phase gazeuse en présence d'un composé de phosphore liquide dans les conditions de réactions, la quantité de phosphore (désignée P), rapportée à la quantité totale d'alcool, étant de 0,01 à 10 ppm.

5. Procédé selon la revendication 1, caractérisé par le fait que l'on utilise, comme catalyseur au cuivre, des corps moulés en cuivre à teneur en cuivre d'au moins 90% en poids.

6. Procédé selon la revendication 1, caractérisé par le fait que l'on utilise, comme catalyseur au cuivre, un catalyseur en coquille en un matériau support inerte revêtu d'une masse active qui est constituée de plus de 90% en poids de cuivre.

7. Procédé selon la revendication 1, caractérisé par le fait que l'on utilise, comme catalyseur à l'argent, un catalyseur en coquille en un matériau support inerte revêtu d'une masse qui est constituée de plus de 90% en poids d'argent.

**Claims**

1. A process for the preparation of a carbonyl compound of the formula

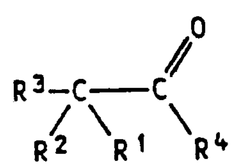

I

where $R^1$ is hydrogen or, together with $R^2$, is oxygen, $R^2$ is $OR^5$ or, together with $R^1$, is oxygen, $R^3$ is hydrogen, alkyl having from 1 to 8 carbon atoms or cycloalkyl, $R^4$ is hydrogen or alkyl having from 1 to 3 carbon atoms, and $R^5$ is cycloalkyl, alkyl having from 1 to 4 carbon atoms or alkoxyalkyl, which may contain a keto or aldehyde moiety, by oxidizing an alcohol of the formula

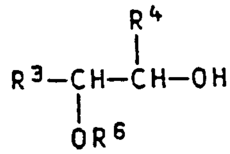

II

where $R^3$ and $R^4$ are as defined above and $R^6$ is hydrogen, alkyl or hydroxyalkyl having from 1 to 4 carbon atoms, cycloalkyl or alkoxyalkyl, which may contain a hydroxyl moiety, in the gas phase using an oxygen-containing gas in the presence of copper- and silver-containing catalysts, which comprises passing the gaseous starting mixture at from 300 to 400°C first over a copper catalyst whose active material has a copper content of greater than 90% by weight, and then over a silver catalyst whose active material has a silver content of greater than 90% by weight, the residence time of the reaction gases in the reactor being from 0.5 to 3 seconds.

2. A process as claimed in claim 1, wherein the alcohol of the formula II is one of the compounds of the formulae

$HOCH_2-CH_2OH$, $H_3COCH_2-CH_2OH$, $C_2H_5OCH_2-CH_2OH$,

$H_3C-CH(OH)CH_2-OH$, $C_2H_5-CH(OH)-CH_2OH$, 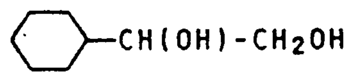 ,

$HO-(CH_2)_2-O-(CH_2)_2-OH$, 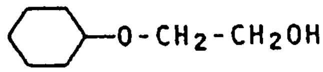 , 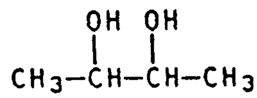

3. A process as claimed in claim 1, wherein glyoxal is prepared from ethylene glycol.

4. A process as claimed in claim 1, wherein the gas-phase oxidation is carried out in the presence of a phosphorus compound which is volatile under the reaction conditions, the amount of phosphorus (calculated P), based on the weight of the alcohol, being from 0.01 to 10 ppm.

5. A process as claimed in claim 1, wherein the copper catalyst employed is in the form of copper moldings containing 90% by weight or more of copper.

6. A process as claimed in claim 1, wherein the copper catalyst used is a shell catalyst comprising an inert carrier material and the active material applied thereto, the latter comprising more than 90% by weight of copper.

7. A process as claimed in claim 1, wherein the silver catalyst used is a shell catalyst comprising an inert carrier material and the active material applied thereto, the latter comprising more than 90% by weight of silver.